# EUROPEAN PATENT SPECIFICATION

(11) **EP 4 126 237 B1**
(45) Date of publication and mention of the grant of the patent: **10.04.2024**
(21) Application number: 21713415.4
(22) Date of filing: 24.03.2021
(51) Int. Cl.: A61P 35/00, C07D 401/04, A61K 31/4178

(54) **DIMALEATE FORM OF 1-((2R,4R)-2-(1H-BENZO[D]IMIDAZOL-2-YL)-1-METHYLPIPERIDIN-4-YL)-3-(4-CYANOPHENYL)UREA**
DIMALEATFORM VON 1-((2R,4R)-2-(1H-BENZO[D]IMIDAZOL-2-YL)-1-METHYLPIPERIDIN-4-YL)-3-(4-CYANOPHENYL)HARNSTOFF
FORME DIMALÉATE DE 1-((2R,4R)-2-(1H-BENZO[D]IMIDAZOL-2-YL)-1-MÉTHYLPIPÉRIDINE?-4-YL)-3-(4-CYANOPHÉNYL)URÉE

(30) Priority: 26.03.2020 EP 20165898
(43) Date of publication of application: 08.02.2023
(73) Proprietor: LEK Pharmaceuticals d.d., 1526 Ljubljana (SI)
(72) Inventor: PEKLAR, Boris, 1526 Ljubljana (SI)
(74) Representative: Prüfer & Partner mbB Patentanwälte · Rechtsanwälte
(86) International application number: PCT/EP2021/057575
(87) International publication number: WO 2021/191278

(56) References cited:
- WO-A1-2016/170451
- WO-A2-2009/004427
- MICHAEL J. MUNCHHOF ET AL: "Discovery of PF-04449913, a Potent and Orally Bioavailable Inhibitor of Smoothened", ACS MEDICINAL CHEMISTRY LETTERS, vol. 3, no. 2, 9 February 2012 (2012-02-09), pages 106-111, XP055207149, ISSN: 1948-5875, DOI: 10.1021/ml2002423 cited in the application
- PEKLAR BORIS ET AL: "Glasdegib Dimaleate: Synthesis, Characterization and Comparison of Its Properties with Monomaleate Analogue", PHARMACEUTICS, vol. 14, no. 8, 6 August 2022 (2022-08-06) , page 1641, XP093090725, DOI: 10.3390/pharmaceutics14081641

## Description

### FIELD OF THE INVENTION

The present invention relates to a dimaleate form of 1-((2*R*,4*R*)-2-(1*H*-benzo[d]imidazol-2-yl)-1-methylpiperidin-4-yl)-3-(4-cyanophenyl)urea (INN: glasdegib) and a process of producing the same. Furthermore, the invention relates to a pharmaceutical composition comprising the the dimaleate form of glasdegib and at least one pharmaceutically acceptable excipient. The pharmaceutical composition of the present invention can be used as a medicament, in particular for the treatment and/or prevention of cancers such as acute myeloid leukemia.

### BACKGROUND OF THE INVENTION

Glasdegib is an inhibitor of the smoothened receptor (Smo), a component of the hedgehog (Hh) signaling pathway that is a potential therapeutic target in a number of human cancers, in particular hematologic malignancies including acute myeloid leukemia (AML), acute lymphoblastic leukemia (ALL), chronic myelomonocytic leukemia (CMML), myelofibrosis (MF) and myelodysplastic syndromes (MDS).

Chemically also designated 1-((2*R*,4*R*)-2-(1*H*-benzo[d]imidazol-2-yl)-1-methylpiperidin-4-yl)-3-(4-cyanophenyl)urea, glasdegib can be represented by the chemical structure as depicted in Formula (I)

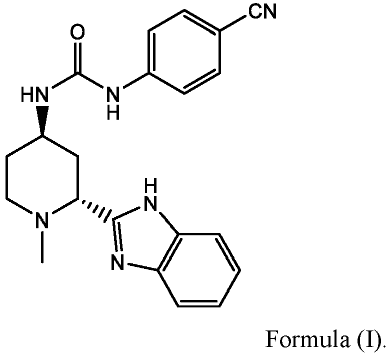

The preparation of glasdegib free base is described in WO 2009/004427 A2 (Example 31). The preparation of glasdegib as a dihydrochloride monohydrate salt has been described by Munchhof et al. (Med. Chem., Lett, 2012, 3:106-111). WO 2016/170451 A1 describes a crystalline glasdegib maleate salt designated as Form 1. According to the teaching of WO 2016/170451 A1, a crystalline glasdegib imidazol complex as well as a crystalline glasdegib (S)-mandelate salt can be used as intermediates for the preparation of the glasdegib maleate salt Form 1. The marketed product Daurismo^{™} contains the glasdedib maleate salt which can be represented by the chemical structure as depicted in Formula (II)

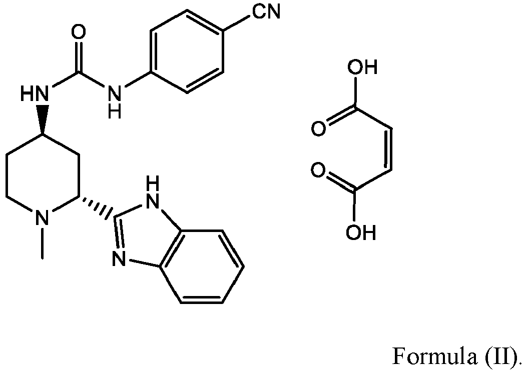

Different solid-state forms of an active pharmaceutical ingredient (API) often possess different physical and chemical properties such as but not limited to dissolution rate, solubility, chemical stability, physical stability, hygroscopicity, melting point, morphology, flowability, bulk density and compressibility. Differences in physicochemical properties of solid-state forms can play a crucial role for the improvement of pharmaceutical compositions, for example, pharmaceutical formulations with improved dissolution profile and bioavailability or with improved stability or shelf-life can become accessible due to an improved solid-state form of an API. Also processing or handling of API during the formulation process may be improved. New solid-state forms of an API can thus have desirable processing properties. They can be easier to handle, better suited for storage, and/or allow for better purification, compared to previously known solid-state forms.

It is thus an objective of the present invention to provide an improved solid-state form of glasdegib.

### SUMMARY OF THE INVENTION

The inventors of the present invention have surprisingly found that when applying very specific crystallization conditions, a crystalline dimaleate form of glasdegib can be obtained.

The crystalline glasdegib dimaleate form of the present invention possesses one or more improved physicochemical properties selected from the group consisting of dissolution rate, solubility, chemical stability, physical stability, hygroscopicity, melting point, morphology, flowability, wettability, bulk density and compressibility.

### Abbreviations

- PXRD: powder X-ray diffractogram
- DSC: differential scanning calorimetry
- RT: room temperature
- RH: relative humidity
- API: active pharmaceutical ingredient

### Definitions

As used herein the term "room temperature" refers to a temperature in the range of from 20 to 30°C.

The term "glasdegib maleate" as used herein refers to the acid addition salt of 1-((2*R*,4*R*)-2-(1*H*-benzo[d]imidazol-2-yl)-1-methylpiperidin-4-yl)-3-(4-cyanophenyl)urea with maleic acid according to the chemical structure depicted in Formula (II) disclosed herein above, wherein glasdegib and maleic acid interact ionically.

The terms "glasdegib dimaleate", "glasdegib dimaleate form" or "dimaleate form of glasdegib" as interchangeably used herein refer to a crystalline compound comprising two molecules maleic acid per molecule glasdegib. The glasdegib dimaleate form of the present invention can be characterized by the chemical structure as depicted in Formula (III) hereinafter. Thereby, the interaction between glasdegib free base and the maleic acid molecules can be ionic (*e.g.* salt-like) and/or non-ionic (*e.g*. co-crystal like).

The term "co-crystal" as used herein refers to crystalline materials composed of two or more different molecular and/or ionic compounds in the same crystal lattice that are associated by nonionic and noncovalent bonds, wherein at least two of the individual molecular and/or ionic compounds are solids at room temperature. Co-crystals are structurally readily distinguishable from salts because unlike salts, their components are in a neutral state and interact nonionically.

The term "glasdegib maleate Form 1" as used herein, refers to the crystalline form of glasdegib maleate, which is disclosed in WO 2016/170451 A2. Form 1 of glasdegib maleate can be characterized by having a powder X-ray diffractogram comprising reflections at 2-Theta angles of (11.6 ± 0.2)°, (12.1 ± 0.2)° and (19.6 ± 0.2)°, when measured at a temperature in the range of from 20 to 30°C with Cu-Kalpha_{1,2} radiation having a wavelength of 0.15419 nm.

As used herein, the term "measured at a temperature in the range of from 20 to 30°C" refers to a measurement under standard conditions. Typically, standard conditions mean a temperature in the range of from 20 to 30°C, *i.e.* at room temperature. Standard conditions can mean a temperature of about 22°C. Typically, standard conditions can additionally mean a measurement under 20-50% relative humidity.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 5.5° 2-Theta for example can appear between 5.3° and 5.7° 2-Theta, preferably between 5.4 and 5.6° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "solid-state form" as used herein refers to any crystalline and/or amorphous phase of a compound. Crystalline phases include anhydrous/non-solvated forms of a compound and their polymorphs, hydrates and solvates of a compound and their polymorphs, salts and co-crystals of a compound and their polymorphs and pseudopolymorphic forms and any mixtures thereof.

As used herein, the term "essentially free of any other solid-state form" with reference to the composition comprising the glasdegib dimaleate form of the present invention, means that the glasdegib dimaleate form contains at most 20 weight%, preferably at most 10 weight%, more preferably at most 5 weight%, 4 weight%, 3 weight%, 2 weight% or 1 weight% of any other solid-state form of glasdegib, based on the total weight of the composition.

The dimaleate form of glasdegib of the present invention may be referred to herein as being characterized by a powder X-ray diffractogram "as shown in" a figure. The person skilled in the art understands that factors such as variations in instrument type, response and variations in sample directionality, sample concentration, sample purity, sample history and sample preparation may lead to variations, for example relating to the exact reflection positions and intensities. However, a comparison of the graphical data in the figure herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid from said solution.

A "predetermined amount" as used herein with regard to the glasdegib dimaleate form of the present invention refers to the initial amount of the glasdegib dimaleate form used for the preparation of a pharmaceutical composition having a desired dosage strength of glasdegib.

As used herein, the term "effective amount" in conjunction with the glasdegib dimaleate form of the present invention encompasses an amount of the glasdegib dimaleate form which causes the desired therapeutic or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient. Excipients may take the function of vehicle, diluent, release agent, disintegrating agent, dissolution modifying agent, absorption enhancer, wetting agent, stabilizer or a manufacturing aid among others.

### BRIEF DESCRIPTION OF THE FIGURES

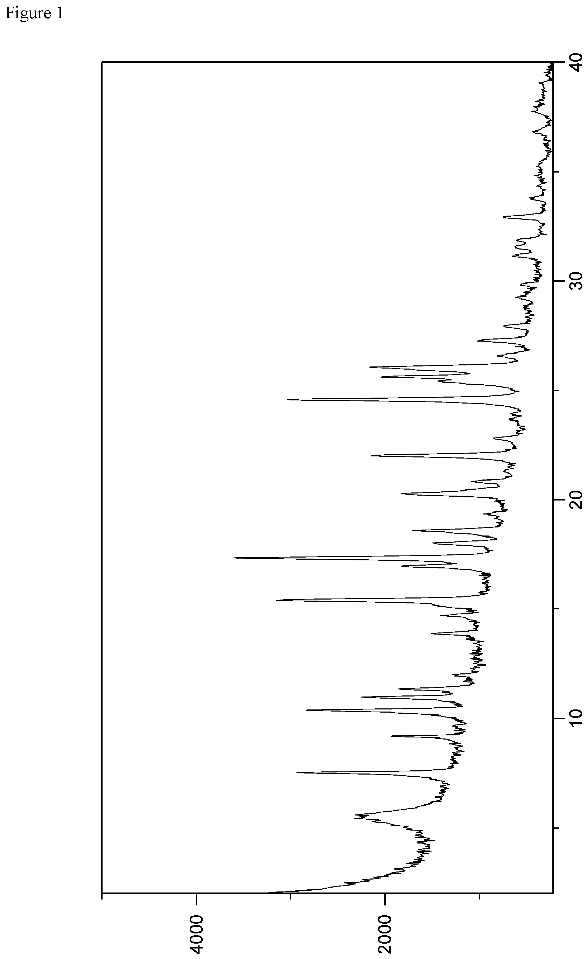

Figure 1: illustrates a representative PXRD of the glasdegib dimaleate form according to the present invention. The x-axis shows the scattering angle in °2-Theta, the y-axis shows the intensity of the scattered X-ray beam in counts of detected photons.

### DETAILED DESCRIPTION OF THE INVENTION

The present invention provides a crystalline dimaleate form of glasdegib.

The crystalline glasdegib dimaleate form of the present invention may be characterized by analytical methods well known in the field of the pharmaceutical industry for characterizing crystalline solids. Such methods comprise but are not limited to powder X-ray diffraction, differential scanning calorimetry and thermogravimetric analysis. The glasdegib dimaleate form of the present invention may be characterized by one of the aforementioned analytical methods or by combining two or more of them. In particular, the dimaleate form of glasdegib of the present invention may be characterized by any one of the following embodiments or by combining two or more of the following embodiments.

In a first aspect, the invention relates to crystalline glasdegib dimaleate.

In one embodiment, the invention relates to crystalline glasdegib dimaleate characterized by having the chemical structure as depicted in Formula (III) 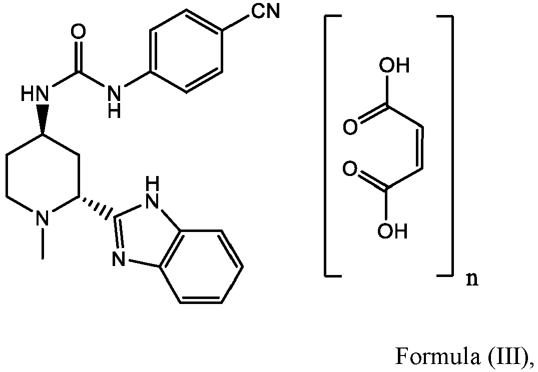 wherein n is in the range of from 1.8 to 2.2, preferably of from 1.9 to 1.1, even more preferably of from 0.95 to 1.05 and most preferably n is 1.0.

In a particular embodiment, the invention relates to crystalline glasdegib dimaleate characterized in that the crystalline glasdegib dimaleate is present as acid addition salt, in particular as crystalline glasdegib dimaleate salt.

In a particularly preferred embodiment, the invention relates to crystalline glasdegib dimaleate characterized in that the crystalline glasdegib dimaleate is present as a co-crystal comprising glasdegib monomaleate as active pharmaceutical ingredient and maleic acid as co-crystal former in the same crystal lattice, wherein the interaction between the glasdegib monomaleate salt and the maleic acid is of nonionic and noncovalent nature.

In another embodiment the invention relates to a crystalline glasdegib dimaleate form characterized by having a PXRD comprising reflections at 2-Theta angles of:
(5.5 ± 0.2)°, (7.5 ± 0.2)° and (17.3 ± 0.2)°; or
(5.5 ± 0.2)°, (7.5 ± 0.2)°, (15.4 ± 0.2)° and (17.3 ± 0.2)°; or
(5.5 ± 0.2)°, (7.5 ± 0.2)°, (10.3 ± 0.2)°, (15.4 ± 0.2)° and (17.3 ± 0.2)°; or
(5.5 ± 0.2)°, (7.5 ± 0.2)°, (9.2 ± 0.2)°, (10.3 ± 0.2)°, (15.4 ± 0.2)° and (17.3 ± 0.2)°; or
(5.5 ± 0.2)°, (7.5 ± 0.2)°, (9.2 ± 0.2)°, (10.3 ± 0.2)°, (15.4 ± 0.2)°, (17.3 ± 0.2)° and (24.6 ± 0.2)°; or
(5.5 ± 0.2)°, (7.5 ± 0.2)°, (9.2 ± 0.2)°, (10.3 ± 0.2)°, (15.4 ± 0.2)°, (17.3 ± 0.2)°, (22.0 ± 0.2)° and (24.6 ± 0.2)°; or
(5.5 ± 0.2)°, (7.5 ± 0.2)°, (9.2 ± 0.2)°, (10.3 ± 0.2)°, (11.0 ± 0.2)°, (15.4 ± 0.2)°, (17.3 ± 0.2)°, (22.0 ± 0.2)° and (24.6 ± 0.2)°; or
(5.5 ± 0.2)°, (7.5 ± 0.2)°, (9.2 ± 0.2)°, (10.3 ± 0.2)°, (11.0 ± 0.2)°, (11.3 ± 0.2)°, (15.4 ± 0.2)°, (17.3 ± 0.2)°, (22.0 ± 0.2)° and (24.6 ± 0.2)°; or
(5.5 ± 0.2)°, (7.5 ± 0.2)°, (9.2 ± 0.2)°, (10.3 ± 0.2)°, (11.0 ± 0.2)°, (11.3 ± 0.2)°, (15.4 ± 0.2)°, (17.3 ± 0.2)°, (18.0 ± 0.2)°, (22.0 ± 0.2)° and (24.6 ± 0.2)°; or
(5.5 ± 0.2)°, (7.5 ± 0.2)°, (9.2 ± 0.2)°, (10.3 ± 0.2)°, (11.0 ± 0.2)°, (11.3 ± 0.2)°, (15.4 ± 0.2)°, (17.3 ± 0.2)°, (18.0 ± 0.2)°, (18.6 ± 0.2)°, (22.0 ± 0.2)° and (24.6 ± 0.2)°; or
(5.5 ± 0.2)°, (7.5 ± 0.2)°, (9.2 ± 0.2)°, (10.3 ± 0.2)°, (11.0 ± 0.2)°, (11.3 ± 0.2)°, (15.4 ± 0.2)°, (17.3 ± 0.2)°, (18.0 ± 0.2)°, (18.6 ± 0.2)°, (20.3 ± 0.2)°, (22.0 ± 0.2)° and (24.6 ± 0.2)°;
when measured at a temperature in the range of from 20 to 30°C with Cu-Kalpha_{1,2} radiation having a wavelength of 0.15419 nm.

In a further embodiment the invention relates to a crystalline glasdegib dimaleate form characterized by having a PXRD comprising reflections at 2-Theta angles of:
(5.5 ± 0.1)°, (7.5 ± 0.1)° and (17.3 ± 0.1)°; or
(5.5 ± 0.1)°, (7.5 ± 0.1)°, (15.4 ± 0.1)° and (17.3 ± 0.1)°; or
(5.5 ± 0.1)°, (7.5 ± 0.1)°, (10.3 ± 0.1)°, (15.4 ± 0.1)° and (17.3 ± 0.1)°; or
(5.5 ± 0.1)°, (7.5 ± 0.1)°, (9.2 ± 0.1)°, (10.3 ± 0.1)°, (15.4 ± 0.1)° and (17.3 ± 0.1)°; or
(5.5 ± 0.1)°, (7.5 ± 0.1)°, (9.2 ± 0.1)°, (10.3 ± 0.1)°, (15.4 ± 0.1)°, (17.3 ± 0.1)° and (24.6 ± 0.1)°; or
(5.5 ± 0.1)°, (7.5 ± 0.1)°, (9.2 ± 0.1)°, (10.3 ± 0.1)°, (15.4 ± 0.1)°, (17.3 ± 0.1)°, (22.0 ± 0.1)° and (24.6 ± 0. 1)°; or
(5.5 ± 0.1)°, (7.5 ± 0.1)°, (9.2 ± 0.1)°, (10.3 ± 0.1)°, (11.0 ± 0.1)°, (15.4 ± 0.1)°, (17.3 ± 0.1)°, (22.0 ± 0.1)° and (24.6 ± 0.1)°; or
(5.5 ± 0.1)°, (7.5 ± 0.1)°, (9.2 ± 0.1)°, (10.3 ± 0.1)°, (11.0 ± 0.1)°, (11.3 ± 0.1)°, (15.4 ± 0.1)°, (17.3 ± 0.1)°, (22.0 ± 0.1)° and (24.6 ± 0.1)°; or
(5.5 ± 0.1)°, (7.5 ± 0.1)°, (9.2 ± 0.1)°, (10.3 ± 0.1)°, (11.0 ± 0.1)°, (11.3 ± 0.1)°, (15.4 ± 0.1)°, (17.3 ± 0.1)°, (18.0 ± 0.1)°, (22.0 ± 0.1)° and (24.6 ± 0.1)°; or
(5.5 ± 0.1)°, (7.5 ± 0.1)°, (9.2 ± 0.1)°, (10.3 ± 0.1)°, (11.0 ± 0.1)°, (11.3 ± 0.1)°, (15.4 ± 0.1)°, (17.3 ± 0.1)°, (18.0 ± 0.1)°, (18.6 ± 0.1)°, (22.0 ± 0.1)° and (24.6 ± 0.1)°; or
(5.5 ± 0.1)°, (7.5 ± 0.1)°, (9.2 ± 0.1)°, (10.3 ± 0.1)°, (11.0 ± 0.1)°, (11.3 ± 0.1)°, (15.4 ± 0.1)°, (17.3 ± 0.1)°, (18.0 ± 0.1)°, (18.6 ± 0.1)°, (20.3 ± 0.1)°, (22.0 ± 0.1)° and (24.6 ± 0.1)°;
when measured at a temperature in the range of from 20 to 30°C with Cu-Kalpha_{1,2} radiation having a wavelength of 0.15419 nm.

In another embodiment the present invention relates to a crystalline glasdegib dimaleate form characterized by having a PXRD comprising reflections at 2-Theta angles of (7.5 ± 0.2)°, (10.3 ± 0.2)°, (11.0 ± 0.2)°, (15.4 ± 0.2)°, (17.3 ± 0.2)°, (20.3 ± 0.2)°, (22.0 ± 0.2)°, (24.6 ± 0.2)°, (25.6 ± 0.2)° and (26.0 ± 0.2)°, when measured at a temperature in the range of from 20 to 30°C with Cu-Kalpha_{1,2} radiation having a wavelength of 0.15419 nm.

In an additional embodiment the invention relates to a crystalline glasdegib dimaleate form characterized by having a PXRD comprising reflections at 2-Theta angles of (7.5 ± 0.1)°, (10.3 ± 0.1)°, (11.0 ± 0.1)°, (15.4 ± 0.1)°, (17.3 ± 0.1)°, (20.3 ± 0.1)°, (22.0 ± 0.1)°, (24.6 ± 0.1)°, (25.6 ± 0.1)° and (26.0 ± 0.1)°, when measured at a temperature in the range of from 20 to 30°C with Cu-Kalpha_{1,2} radiation having a wavelength of 0.15419 nm.

In another embodiment the invention relates to a crystalline glasdegib dimaleate form characterized by having a PXRD essentially the same as shown in Figure 1 of the present invention, when measured at a temperature in the range of from 20 to 30°C with Cu-Kalpha_{1,2} radiation having a wavelength of 0.15419 nm.

In yet another embodiment, the present invention relates to a crystalline glasdegib dimaleate form characterized by having a DSC curve comprising an endothermic peak having an onset at a temperature of (166 ± 5)°C, preferably of (166 ± 3)°C, more preferably of (166 ± 2)°C and most preferably of (166 ± 1)°C, when measured at a heating rate of 10 K/min.

In a further embodiment, the present invention relates to a crystalline glasdegib dimaleate form characterized by having a DSC curve comprising an endothermic peak having a maximum at a temperature of (169 ± 5)°C, preferably of (169 ± 3)°C, more preferably of (169 ± 2)°C and most preferably of (169 ± 1)°C, when measured at a heating rate of 10 K/min.

In another aspect, the present invention relates to a composition comprising the crystalline glasdegib dimaleate form of the present invention as defined in any one of the above described embodiments, said composition being essentially free of any other solid-state form of glasdegib. For example, a composition comprising the glasdegib dimaleate form of the present invention comprises at most 20 weight%, preferably at most 10 weight%, more preferably at most 5 weight%, 4 weight%, 3 weight%, 2 weight% or 1 weight% of any other solid-state form of glasdegib, based on the total weight of the composition. Preferably, the any other solid-state form of glasdegib is glasdegib maleate Form I of WO 2016/170451 A2.

In a further aspect, the present invention relates to a process for the preparation of the glasdegib dimaleate form of the present invention or the composition comprising the glasdegib dimaleate form as defined in any one of the above described aspects and their corresponding embodiments comprising:
(a) dissolving glasdegib maleate and maleic acid in a solvent comprising dichloromethane, methanol or a mixture thereof, wherein the molar ratio of glasdegib maleate and maleic acid is in the range of from 1.0 : 0.5 to 1.0 : 3.0 (glasdegib maleate : maleic acid);
(b) optionally filtering the solution obtained in (a) in order to remove any undissolved solid;
(c) removing at least a part of the solvent in order to initiate crystallization;
(d) separating at least a part of the crystals obtained in (c) from the mother liquor; and
(e) drying the crystals obtained in step (c) or (d).

Glasdegib free base can for example be prepared according to the procedure provided in Example 31 of WO 2009/004427 A2. Glasdegib maleate can be prepared by reacting glasdegib free base with maleic acid in a suitable solvent. For example, glasdegib maleate can be prepared according to the procedure provided in Example 2 of WO 2016/170451 A1.

Glasdegib maleate, which is used as starting material in step (a) of the above described process, may be applied as crystalline and/or amorphous material. In a first step, glasdegib maleate and maleic acid are dissolved in the applied solvent, preferably at increased temperature, more preferably at increased temperature and with applying sonication. Suitable solvents which can be used are selected from the group consisting of dichlormethane, methanol or a mixture composed of dichlormethane and methanol. The applied glasdegib maleate concentration depends on the solvent or solvent mixture used and may be in the range of from about 0.1 to 50 g/L, preferably of from about 5 to 20 g/L. The molar ratio of glasdegib maleate and maleic acid applied is in the range of from about 1.0 : 0.5 to 1.0 : 3.0 (glasdegib maleate : maleic acid), preferably of from about 1.0 : 0.6 to 1.0 to 1.6 (glasdegib maleate : maleic acid) and most preferably of from about 1.0 : 1.0 to 1.0 to 1.2 (glasdegib maleate : maleic acid).

In order to initiate crystallization the solution is partially concentrated, *e.g*. by slow solvent evaporation, preferably under stirring. Optionally, glasdegib dimaleate seed crystals may be added in order to promote crystallization and/or to control particle size distribution. The amount of seed crystals employed may range from about 1 to 20 weight%, preferably from about 1 to 10 weight% and most preferably from about 1 to 5 weight%, based on the weight of applied glasdegib maleate starting material. Seed crystals may be prepared according to Examples 1 to 3 hereinafter.

Once the glasdegib dimaleate form of the present invention is obtained or preferably obtained in essentially pure form, at least a part of the crystals is separated from the mother liquor. Preferably, the crystals are separated from their mother liquor by any conventional method such as filtration, centrifugation, solvent evaporation or decantation, more preferably by filtration or centrifugation and most preferably by filtration.

Optionally, in a further step the isolated crystals are washed with a solvent selected from the group consisting of dichlormethane, methanol or a mixture composed of dichlormethane and methanol.

In a final step, the obtained crystals are dried. Drying may be performed at a temperature in the range of from about 20 to 80°C, preferably in the range of from about 20 to 40°C and most preferably drying is performed at RT. Drying may be performed for a period in the range of from about 1 to 72 hours, preferably of from about 2 to 48 hours, more preferably of from about 4 to 24 hours and most preferably of from about 6 to 18 hours. Drying may be performed at ambient pressure and/ or under reduced pressure. Preferably, drying is performed at a pressure of about 100 mbar or less, more preferably of about 50 mbar or less.

In a further aspect the present invention relates to the use of the glasdegib dimaleate form of the present invention or the composition comprising the glasdegib dimaleate form as defined in any one of the above described aspects and their corresponding embodiments for the preparation of a pharmaceutical composition.

In a further aspect, the present invention relates to a pharmaceutical composition comprising the glasdegib dimaleate form of the present invention or the composition comprising the glasdegib dimaleate form as defined in any one of the above described aspects and their corresponding embodiments, preferably in an effective and/or predetermined amount, and at least one pharmaceutically acceptable excipient.

Preferably, the predetermined and/or effective amount of the glasdegib dimaleate form of the present invention is in the range of from 20 to 100 mg calculated as glasdegib free base. For example the predetermined and/or effective amount of the glasdegib dimaleate form of the present invention is selected from the group consisting of 20 mg, 25 mg, 50 mg and 100 mg calculated as glasdegib free base. In a particular preferred embodiment the predetermined and/or effective amount of the glasdegib dimaleate form of the present invention is 25 mg or 100 mg, calculated as glasdegib free base.

Preferably, the pharmaceutical composition of the present invention as described above is an oral solid dosage form. In a particular embodiment the pharmaceutical composition of the present invention as describe above is a tablet, preferably a film-coated tablet comprising a tablet core and a coating.

The tablet or tablet core may be prepared by mixing the glasdegib dimaleate form of the present invention with at least one pharmaceutically acceptable excipient followed by compressing the mixture. Optionally, a granulation step such as a wet or dry granulation step is performed before compression. Preferably, the tablet core is subsequently coated with a film-coat. Methods of preparing such tablets, tablet cores and film-coated tablets are well known in the pharmaceutical arts.

In a further aspect, the present invention relates to the glasdegib dimaleate form, the composition comprising the glasdegib dimaleate form or the pharmaceutical composition comprising the glasdegib dimaleate form or the composition comprising the glasdegib dimaleate form as defined in any one of the above described aspects and their corresponding embodiments for use as a medicament.

In yet another aspect, the present invention relates to the glasdegib dimaleate form, the composition comprising the glasdegib dimaleate form or the pharmaceutical composition comprising the glasdegib dimaleate form or the composition comprising the glasdegib dimaleate form as defined in any one of the above described aspects and their corresponding embodiments for use in the treatment of cancer.

In an alternative embodiment, the invention concerns the glasdegib dimaleate form for use in a method of treating cancer said method comprising administering an effective amount of the glasdegib dimaleate form, the composition comprising the glasdegib dimaleate form or the pharmaceutical composition comprising the glasdegib dimaleate form or the composition comprising the glasdegib dimaleate form as defined in any one of the above described aspects and their corresponding embodiments to a patient in need of such a treatment.

For example, the cancer is selected from the group consisting of hematologic malignancies including acute myeloid leukemia (AML), acute lymphoblastic leukemia (ALL), chronic myelomonocytic leukemia (CMML), myelofibrosis (MF) and myelodysplastic syndromes (MDS). In a particular preferred embodiment, the cancer is acute myeloid leukemia (AML).

### EXAMPLES

The following non-limiting examples are illustrative for the disclosure and are not to be construed as to be in any way limiting for the scope of the invention.

### Example 1: Preparation of the glasdegib dimaleate form of the present invention

A mixture of glasdegib maleate (100 mg, 0.22 mmol, *e.g*. prepared according to Example 2 of WO 2016/170451 A1), maleic acid (amounts as per Table 1) and dichloromethane (40 mL) was heated to 40°C and sonicated in an ultrasonic bath. Any undissolved solid was removed by filtering the mixture through a 0.45 µm syringe filter into a fresh vial fitted with a magnetic stir bar. The thus obtained solution was stirred at room temperature and a part of the solvent was allowed to evaporate slowly through a small hole on the vial cap over the course of approximately 16 hours. The resulting precipitate was isolated by filtration and dried for about 16 hours at room temperature and under reduced pressure of about 50 mbar.

**Table 1: Amounts of maleic acid used and result obtained according to powder X-ray diffraction**

| **Example** | **Amount maleic acid** | **Result according to powder X-ray diffraction** |
|---|---|---|
| 1.1 | 15 mg (0.13 mmol) | Crystalline glasdegib dimaleate form |
| 1.2 | 25 mg (0.22 mmol) | Crystalline glasdegib dimaleate form |
| 1.3 | 31 mg (0.27 mmol) | Crystalline glasdegib dimaleate form |
| 1.4 | 41 mg (0.35 mmol) | Crystalline glasdegib dimaleate form |

### Example 2: Preparation of the glasdegib dimaleate form of the present invention

Glasdegib maleate (100 mg, 0.22 mmol, e.g. prepared according to Example 2 of WO 2016/170451 A1) and maleic acid (31 mg, 0.27 mmol) were dissolved in a mixture of dichloromethane (1.9 mL) and methanol (0.1 mL) upon heating to 40°C and with the aid of sonication in an ultrasonic bath. The obtained clear solution was filtered through 0.45 µm syringe filter into a fresh vial fitted with a stir bar. Under constant mixing dichloromethane (12 mL) was added to the solution. After 2 hours of stirring a white suspension was formed, which was further stirred at room temperature for approximately 16 hours. The precipitate was isolated by filtration, rinsed with dichloromethane (1 mL) and dried for about 16 hours at room temperature and under reduced pressure of about 50 mbar to obtain the glasdegib dimaleate form of the present invention.

### Example 3: Preparation of the glasdegib dimaleate form of the present invention

Glasdegib maleate (100 mg, 0.22 mmol, e.g. prepared according to Example 2 of WO 2016/170451 A1) and maleic acid (31 mg, 0.27 mmol) were dissolved in methanol (6 mL) upon heating to 60°C with the aid of sonication in an ultrasonic bath. The obtained clear solution was filtered through a 0.45 µm syringe filter into fresh vial fitted with a stir bar. The thus obtained solution was stirred at room temperature and a part of the solvent was allowed to evaporate slowly through a small hole on the vial cap over the course of approximately 3 days. The resulting precipitate was isolated by filtration and dried for about 16 hours at room temperature and under reduced pressure of about 50 mbar to obtain the glasdegib dimaleate form of the present invention.

### Example 4: Powder X-ray diffraction

The glasdegib dimaleate form according to the present invention was investigated by powder X-ray diffraction, which was performed on a PANalytical Empyrean diffractometer equipped with a theta/theta coupled goniometer in transmission geometry, Cu-Kα1,2 radiation (wavelength 0.15419 nm) with a focusing mirror and a solid state PIXcel1D detector. The patterns were recorded at a tube voltage of 45 kV and a tube current of 40 mA, applying a stepsize of 0.026° 2-Theta with 50s per step in the angular range of 2° to 40° 2-Theta at ambient conditions. A typical precision of the 2-theta values is in the range of about ± 0.2° 2-Theta, preferably of about ± 0.1° 2-Theta . Thus a diffraction peak that appears at 5.5° 2-Theta can appear between 5.3 and 5.7° 2-Theta, preferably between 5.4 and 5.6° 2-Theta on most X-ray diffractometers under standard conditions.

A representative diffractogram of the glasdegib dimaleate form according to the present invention is displayed in Figure 1 and the corresponding reflection list (peak list) from 2 to 30° 2-Theta is provided in Table 2 below.

**Table 2: Reflection (peak) positions of the glasdegib dimaleate form according to the present invention in the range of from 2 to 30° 2-Theta; A typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 5.5 | 13.8 | 19.3 | 25.6 |
| 7.5 | 14.7 | 20.3 | 26.0 |
| 9.2 | 15.4 | 20.8 | 26.6 |
| 10.3 | 17.0 | 22.0 | 27.3 |
| 11.0 | 17.3 | 22.8 | 27.9 |
| 11.3 | 18.0 | 24.6 | 29.2 |
| 11.9 | 18.6 | 25.4 | 29.8 |

### Example 5: Differential scanning calorimetry (DSC)

The glasdegib dimaleate form according to the present invention was investigated by DSC, which was performed on a Mettler Toledo DSC 3+ STAR system. The sample (0.5470 mg) was heated in a 40 microliter aluminium pan with a pierced aluminium lid from 30 to 300°C at a rate of 10 K/min. Nitrogen (purge rate 200 mL/min) was used as purge gas.

The DSC curve shows no thermal event until a first endothermic peak with an onset temperature of about 166°C and a peak temperature of about 169°C appears, which indicates the good thermal stability of the glasdegib dimaleate form of the present invention.

## Claims

1. Crystalline glasdegib dimaleate.

2. The crystalline glasdegib dimaleate of claim 1 **characterized by** having a chemical structure as depicted in Formula (III) 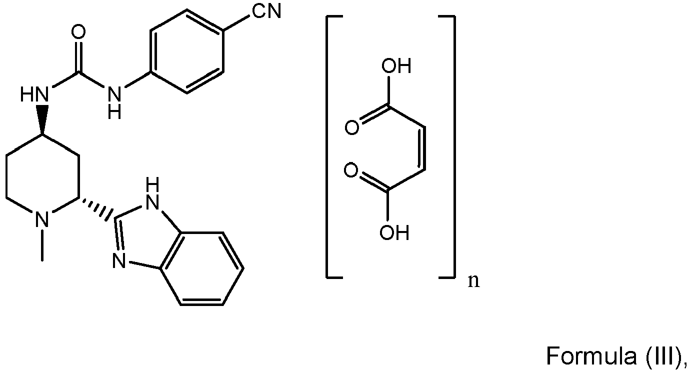 wherein n is in the range of from 1.8 to 2.2.

3. The crystalline glasdegib dimaleate according to claim 1 or 2 **characterized by** having a powder X-ray diffractogram comprising reflections at 2-Theta angles of (5.5 ± 0.2)°, (7.5 ± 0.2)° and (17.3 ± 0.2)°, when measured at a temperature in the range of from 20 to 30°C with Cu-Kalpha_{1,2} radiation having a wavelength of 0.15419 nm.

4. The crystalline glasdegib dimaleate according to claim 3 **characterized by** having a powder X-ray diffractogram comprising additional reflections at 2-Theta angles of (10.3 ± 0.2)° and/or (15.4 ± 0.2)°, when measured at a temperature in the range of from 20 to 30°C with Cu-Kalpha_{1,2} radiation having a wavelength of 0.15419 nm.

5. The crystalline glasdegib dimaleate according to any one of the preceding claims **characterized by** having a DSC curve comprising an endothermic peak having an onset at a temperature of (166 ± 5)°C, when measured at a heating rate of 10 K/min.

6. A composition comprising the crystalline glasdegib dimaleate as defined in any one of the preceding claims **characterized by** comprising at most 20 weight%, preferably at most 10 weight%, more preferably at most 5 weight%, 4 weight%, 3 weight%, 2 weight% or 1 weight% of any other solid-state form of glasdegib, based on the total weight of the composition.

7. The composition of claim 6, wherein the any other solid-state form of glasdegib is glasdegib maleate Form I o**characterized by** having a powder X-ray diffractogram comprising reflections at 2-Theta angles of (11.6 ± 0.2)°, (12.1 ± 0.2)° and (19.6 ± 0.2)°, when measured at a temperature in the range of from 20 to 30°C with Cu-Kalpha_{1,2} radiation having a wavelength of 0.15419 nm.

8. Use of the crystalline glasdegib dimaleate as defined in any one of claims 1 to 5 or the composition as defined in claim 6 or 7 for the preparation of a pharmaceutical composition.

9. A pharmaceutical composition comprising the crystalline glasdegib dimaleate as defined in any one of claims 1 to 5 or the composition as defined in claim 6 or 7 and at least one pharmaceutically acceptable excipient.

10. The pharmaceutical composition according to claim 9, wherein the pharmaceutical composition is an oral solid dosage form.

11. The pharmaceutical composition of claim 10, wherein the oral solid dosage form is a tablet.

12. The crystalline glasdegib dimaleate as defined in any one of claims 1 to 5, the composition as defined in claim 6 or 7 or the pharmaceutical composition according to any one of claims 9 to 11 for use as a medicament.

13. The crystalline glasdegib dimaleate as defined in any one of claims 1 to 5, the composition as defined in claim 6 or 7 or the pharmaceutical composition according to any one of claims 9 to 11 for use in the treatment of cancer.

14. The crystalline glasdegib dimaleate, the composition, or the pharmaceutical composition for use according to claim 13, wherein the cancer is selected from the group consisting of hematologic malignancies including acute myeloid leukemia (AML), acute lymphoblastic leukemia (ALL), chronic myelomonocytic leukemia (CMML), myelofibrosis (MF) and myelodysplastic syndromes (MDS).

15. A process for the preparation of the crystalline glasdegib dimaleate as defined in any one of claims 1 to 5 or the composition as defined in claim 6 or 7 comprising:
(a) dissolving glasdegib maleate and maleic acid in a solvent comprising dichloromethane, methanol or a mixture thereof, wherein the molar ratio of glasdegib maleate and maleic acid is in the range of from 1.0 : 0.5 to 1.0 : 3.0 (glasdegib maleate : maleic acid);
(b) optionally filtering the solution obtained in (a) in order to remove any undissolved solid;
(c) removing at least a part of the solvent in order to initiate crystallization;
(d) separating at least a part of the crystals obtained in (c) from the mother liquor; and
(e) drying the crystals obtained in step (c) or (d).

## Patentansprüche

1. Kristallines Glasdegib-Dimaleat.

2. Kristallines Glasdegib-Dimaleat nach Anspruch 1, **gekennzeichnet durch** das Aufweisen einer chemischen Struktur wie in Formel (III) dargestellt 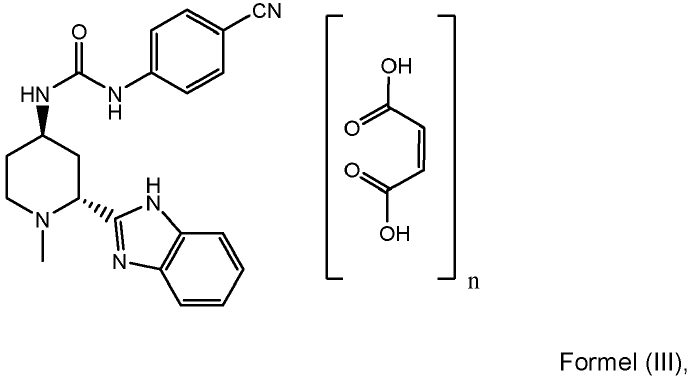 wobei n im Bereich von 1,8 bis 2,2 liegt.

3. Kristallines Glasdegib-Dimaleat nach Anspruch 1 oder 2, **gekennzeichnet durch** ein Pulver-Röntgendiffraktogramm, das Reflexe bei 2-Theta-Winkeln von (5,5 ± 0,2)°, (7,5 ± 0,2)° und (17,3 ± 0,2)° umfasst, wenn es bei einer Temperatur im Bereich von 20 bis 30°C mit Cu-Kalpha_{1,2}-Strahlung mit einer Wellenlänge von 0,15419 nm gemessen wird.

4. Kristallines Glasdegib-Dimaleat nach Anspruch 3, **gekennzeichnet durch** ein Pulver-Röntgendiffraktogramm, das zusätzliche Reflexe bei 2-Theta-Winkeln von (10,3 ± 0,2)° und/oder (15,4 ± 0,2)° umfasst, wenn es bei einer Temperatur im Bereich von 20 bis 30°C mit Cu-Kalpha_{1,2}-Strahlung mit einer Wellenlänge von 0,15419 nm gemessen wird.

5. Kristallines Glasdegib-Dimaleat nach irgendeinem der vorhergehenden Ansprüche, **gekennzeichnet durch** eine DSC-Kurve, die einen endothermen Peak mit einem Beginn bei einer Temperatur von (166 ± 5)°C umfasst, wenn sie bei einer Heizrate von 10 K/min gemessen wird.

6. Zusammensetzung, umfassend das kristalline Glasdegib-Dimaleat, wie in irgendeinem der vorhergehenden Ansprüche definiert, **gekennzeichnet durch** Umfassen von höchstens 20 Gew.-%, bevorzugt höchstens 10 Gew.-%, noch bevorzugter von höchstens 5 Gew.-%, 4 Gew.-%, 3 Gew.-%, 2 Gew.-% oder 1 Gew.-% jeglicher anderen Festkörperform von Glasdegib, bezogen auf das Gesamtgewicht der Zusammensetzung.

7. Zusammensetzung nach Anspruch 6, wobei die jegliche andere Festkörperform von Glasdegib Glasdegib-Maleat Form I ist, **gekennzeichnet durch** ein Pulver-Röntgendiffraktogramm, das Reflexe bei 2-Theta-Winkeln von (11,6 ± 0,2)°, (12,1 ± 0,2)° und (19,6 ± 0,2)° umfasst, gemessen bei einer Temperatur im Bereich von 20 bis 30°C mit Cu-Kalpha_{1,2}-Strahlung mit einer Wellenlänge von 0,15419 nm.

8. Verwendung des kristallinen Glasdegib-Dimaleats, wie in einem der Ansprüche 1 bis 5 definiert, oder der Zusammensetzung, wie in Anspruch 6 oder 7 definiert, zur Herstellung einer pharmazeutischen Zusammensetzung.

9. Pharmazeutische Zusammensetzung, umfassend das kristalline Glasdegib-Dimaleat, wie in einem der Ansprüche 1 bis 5 definiert, oder die Zusammensetzung, wie in Anspruch 6 oder 7 definiert, und mindestens einen pharmazeutisch akzeptablen Hilfsstoff.

10. Pharmazeutische Zusammensetzung nach Anspruch 9, wobei die pharmazeutische Zusammensetzung eine orale feste Darreichungsform ist.

11. Pharmazeutische Zusammensetzung nach Anspruch 10, wobei die orale feste Darreichungsform eine Tablette ist.

12. Kristallines Glasdegib-Dimaleat wie in irgendeinem der Ansprüche 1 bis 5 definiert, Zusammensetzung wie in Anspruch 6 oder 7 definiert oder pharmazeutische Zusammensetzung nach irgendeinem der Ansprüche 9 bis 11, zur Verwendung als Arzneimittel.

13. Kristallines Glasdegib-Dimaleat wie in irgendeinem der Ansprüche 1 bis 5 definiert, Zusammensetzung wie in Anspruch 6 oder 7 definiert oder pharmazeutische Zusammensetzung nach irgendeinem der Ansprüche 9 bis 11, zur Verwendung bei der Behandlung von Krebs.

14. Kristallines Glasdegib-Dimaleat, Zusammensetzung oder pharmazeutische Zusammensetzung zur Verwendung gemäß Anspruch 13, wobei der Krebs ausgewählt ist aus der Gruppe bestehend aus hämatologischen Malignomen einschließlich akuter myeloischer Leukämie (AML), akuter lymphatischer Leukämie (ALL), chronischer myelomonozytischer Leukämie (CMML), Myelofibrose (MF) und myelodysplastischen Syndromen (MDS).

15. Verfahren zur Herstellung des kristallinen Glasdegib-Dimaleats, wie in irgendeinem der Ansprüche 1 bis 5 definiert, oder der Zusammensetzung, wie in Anspruch 6 oder 7 definiert, umfassend:
(a) Auflösen von Glasdegib-Maleat und Maleinsäure in einem Lösungsmittel, das Dichlormethan, Methanol oder eine Mischung davon umfasst, wobei das Molverhältnis von Glasdegib-Maleat und Maleinsäure im Bereich von 1,0 : 0,5 bis 1,0 : 3,0 (Glasdegib-Maleat: Maleinsäure) liegt;
(b) optionales Filtrieren der Lösung, die in (a) erhalten wurde, um irgendwelche ungelösten Feststoffe zu entfernen;
(c) Entfernen mindestens eines Teils des Lösungsmittels, um die Kristallisation zu initiieren;
(d) Abtrennen mindestens eines Teils der Kristalle, die in (c) erhalten wurden, von der Mutterlauge; und
(e) Trocknen der Kristalle, die in Schritt (c) oder (d) erhalten wurden.

## Revendications

1. Dimaléate de glasdegib cristallin.

2. Dimaléate de glasdegib cristallin selon la revendication 1, **caractérisé en ce qu'**il a une structure chimique telle que représentée dans la formule (III) 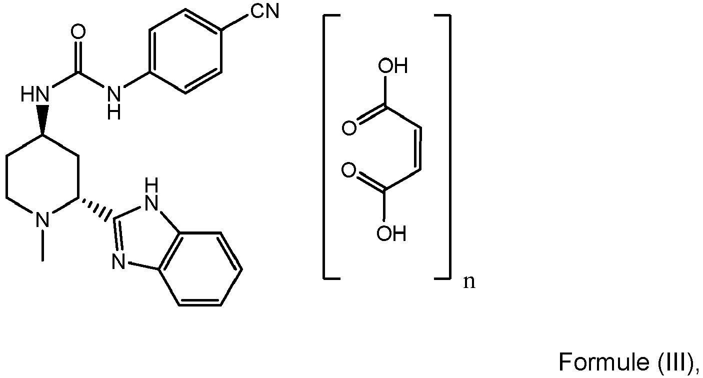 dans laquelle n est dans la plage de 1,8 à 2,2.

3. Dimaléate de glasdegib cristallin selon la revendication 1 ou 2, **caractérisé en ce qu'**il a un diffractogramme de rayons X sur poudre comprenant des réflexions à des angles 2-thêta de (5,5 ± 0,2)°, (7,5 ± 0,2)° et (17,3 ± 0,2)°, lorsqu'elles sont mesurées à une température dans la plage de 20 à 30 °C avec un rayonnement Cu-Kalpha_{1,2} ayant une longueur d'onde de 0,15419 nm.

4. Dimaléate de glasdegib cristallin selon la revendication 3, **caractérisé en ce qu'**il a un diffractogramme de rayons X sur poudre comprenant des réflexions supplémentaires à des angles 2-thêta de (10,3 ± 0,2)° et/ou (15,4 ± 0,2)°, lorsqu'elles sont mesurées à une température dans la plage de 20 à 30 °C avec un rayonnement Cu-Kalpha_{1,2} ayant une longueur d'onde de 0,15419 nm.

5. Dimaléate de glasdegib cristallin selon l'une quelconque des revendications précédentes, **caractérisé en ce qu'**il a une courbe DSC comprenant un pic endothermique ayant un début à une température de (166 ± 5) °C, lorsqu'elle est mesurée à une vitesse de chauffage de 10 K/min.

6. Composition comprenant le dimaléate de glasdegib cristallin tel que défini dans l'une quelconque des revendications précédentes, **caractérisée en ce qu'**elle comprend au plus 20 % en poids, de préférence au plus 10 % en poids, plus préférablement au plus 5 % en poids, 4 % en poids, 3 % en poids, 2 % en poids ou 1 % en poids de toute autre forme à l'état solide de glasdegib, sur la base du poids total de la composition.

7. Composition selon la revendication 6, dans laquelle toute autre forme à l'état solide de glasdegib est le maléate de glasdegib forme I, **caractérisée en ce qu'**elle a un diffractogramme de rayons X sur poudre comprenant des réflexions à des angles 2-thêta de (11,6 ± 0,2)°, (12,1 ± 0,2)° et (19,6 ± 0,2)°, lorsqu'elles sont mesurées à une température dans la plage de 20 à 30 °C avec un rayonnement Cu-Kalpha_{1,2} ayant une longueur d'onde de 0,15419 nm.

8. Utilisation du dimaléate de glasdegib cristallin tel que défini dans l'une quelconque des revendications 1 à 5 ou de la composition telle que définie dans la revendication 6 ou 7 pour la préparation d'une composition pharmaceutique.

9. Composition pharmaceutique comprenant le dimaléate de glasdegib cristallin tel que défini dans l'une quelconque des revendications 1 à 5 ou la composition telle que définie dans la revendication 6 ou 7 et au moins un excipient pharmaceutiquement acceptable.

10. Composition pharmaceutique selon la revendication 9, dans laquelle la composition pharmaceutique est une forme de dosage solide orale.

11. Composition pharmaceutique selon la revendication 10, dans laquelle la forme de dosage solide orale est un comprimé.

12. Dimaléate de glasdegib cristallin tel que défini dans l'une quelconque des revendications 1 à 5, composition telle que définie dans la revendication 6 ou 7 ou composition pharmaceutique selon l'une quelconque des revendications 9 à 11 pour une utilisation en tant que médicament.

13. Dimaléate de glasdegib cristallin tel que défini dans l'une quelconque des revendications 1 à 5, composition telle que définie dans la revendication 6 ou 7 ou composition pharmaceutique selon l'une quelconque des revendications 9 à 11 pour une utilisation dans le traitement du cancer.

14. Dimaléate de glasdegib cristallin, composition, ou composition pharmaceutique pour une utilisation selon la revendication 13, dans lequel le cancer est choisi dans le groupe constitué par les malignités hématologiques incluant la leucémie aiguë myéloïde (LAM), la leucémie aiguë lymphoblastique (LAL), la leucémie myélomonocytaire chronique (CMML), la myélofibrose (MF) et les syndromes myélodysplasiques (MDS).

15. Procédé de préparation du dimaléate de glasdegib cristallin tel que défini dans l'une quelconque des revendications 1 à 5 ou de la composition telle que définie dans la revendication 6 ou 7 comprenant :
(a) dissolver du maléate de glasdegib et de l'acide maléique dans un solvant comprenant du dichlorométhane, du méthanol ou un mélange de ceux-ci, dans lequel le rapport molaire du maléate de glasdegib et de l'acide maléique est dans la plage de 1,0:0,5 à 1,0:3,0 (maléate de glasdegib:acide maléique) ;
(b) éventuellement filtrer la solution obtenue en (a) afin d'éliminer tout solide non dissous ;
(c) éliminer au moins une partie du solvant afin d'initier la cristallisation ;
(d) séparer au moins une partie des cristaux obtenus en (c) de la liqueur mère ; et
(e) sécher les cristaux obtenus dans l'étape (c) ou (d).
